# FASCICULE DE BREVET EUROPEEN

(11) **EP 0 499 502 B1**
(45) Date de publication et mention de la délivrance du brevet: **01.06.1994**
(21) Numéro de dépôt: 92400217.3
(22) Date de dépôt: 28.01.1992
(51) Int. Cl.: C02F 3/28

(54) **Procédé de régulation d'un dispositif de dépollution d'eaux résiduaires**
Verfahren für die Regelung einer Vorrichtung zur Abwasserreinigung
Process for controlling an apparatus for waste water purification

(30) Priorité: 11.02.1991 FR 9101542
(43) Date de publication de la demande: 19.08.1992
(73) Titulaire: DEGREMONT, 92508 Rueil-Malmaison Cédex (FR); INSTITUT NATIONAL DE LA RECHERCHE AGRONOMIQUE, F-75341 Paris Cédex 07 (FR); CENTRE NATIONAL DE LA RECHERCHE SCIENTIFIQUE (CNRS), 75700 Paris Cedex 07 (FR)
(72) Inventeur: Moletta, Renato, F-11180 Le Somail (FR); Ehlinger, Frédéric, F-78230 Le Pecq (FR); Couderc, Jean-Pierre, F-66680 Perpignan (FR)
(74) Mandataire: Armengaud Ainé, Alain

(56) Documents cités:
- US-A- 4 986 916
- JOURNAL OF THE WATER POLLUTION CONTROL FEDERATION. vol. 59, no. 3, Mars 1987,WASHINGTON US pages 152 - 161; HARPER, STEPHEN R. ET AL.: 'ENHANCEMENT OFANAEROBIC TREATMENT EFFICIENCY THROUGH PROCESS MODIFICATION'
- PATENT ABSTRACTS OF JAPAN vol. 6, no. 208 (C-130)(1086) 20 Octobre 1982
- WATER RESEARCH. vol. 24, no. 1, Janvier 1990, OXFORD GB pages 121 - 123; W.R.
- SLATER ET AL.: 'A MICROCOMPUTER-BASED INSTRUMENTATION SYSTEM ETC.'

## Description

La présente invention est relative à un procédé de régulation et de conduite automatique d'un dispositif de dépollution d'eaux résiduaires par fermentation méthanique.

On sait que l'on utilise fréquemment en tant que procédé de dépollution efficace la technique de la fermentation méthanique qui est appliquée en particulier au traitement des effluents agroalimentaires. Les procédés d'épuration de ce type mettent en oeuvre des réacteurs de plus en plus intensifs qui permettent de confiner d'importantes concentrations en biomasse dans des volumes très compacts De ce fait, les écosystèmes sont en permanence fortement sollicités en vue de travailler dans les conditions limites compatibles avec un bon équilibre microbien. L'optimisation du fonctionnement de tels réacteurs devient très difficile et le personnel qui est affecté à l'exploitation d'une station d'épuration anaérobie n'étant pas toujours convenablement formé, il advient que le dispositif de dépollution travaille souvent en deçà de ses possibilités par crainte d'une surcharge organique.

Selon la technique actuelle, le contrôle du fonctionnement des fermenteurs anaérobies dépend de l'évolution de certains paramètres tels que notamment le pH, le pouvoir tampon en carbonates et le taux de dégradation de la demande chimique en oxygène (DCO), ce paramètre étant considéré comme représentatif de la disparition des éléments polluants. Dans ces installations de type connu, la mesure de ces paramètres s'effectue bien entendu sur la phase liquide des réacteurs et par prélèvement discontinu.

Or, compte tenu de la particularité de l'écosystème anaérobie à fonctionner en étapes successives, acidogénèse puis méthanogénèse, le pH et la DCO ne sont pas forcément les paramètres les plus judicieux à mesurer en ce qui concerne la disparition des éléments polluants de l'installation de dépollution.

En effet, dans une première étape, la matière organique est transformée en acides gras volatils par l'action des bactéries acidogènes puis, dans une seconde étape, les acides gras volatils ainsi obtenus sont dégradés en méthane et en gaz carbonique, et de ce fait, ils n'influencent plus le pH du milieu si le pouvoir tampon est suffisant. Par contre, en cas de surcharge organique par exemple, les acides gras volatils peuvent s'accumuler et faire baisser le pH et alors, les mesures de pH et de la DCO traduisent un fait accompli et, il est souvent trop tard pour réagir lorsque les valeurs mesurées de ces deux paramètres atteignent des seuils critiques.

Par ailleurs, le paramètre pH est fiable à la condition toutefois que les sondes qui sont utilisées en vue de sa détermination à partir de la phase liquide du réacteur soient fréquemment et régulièrement entretenues, ce qui constitue bien entendu une mesure souvent astreignante. En outre, les mesures des deux autres paramètres : carbonates et DCO demandent un minimum d'équipement de laboratoire. Compte tenu du fait qu'il convient d'effectuer des prélèvements puis de les analyser, et que le temps d'analyse est très long (d'une durée de l'ordre de 2 heures), on ne fait en général au maximum qu'une seule mesure par jour. Par ailleurs, à l'heure actuelle, une telle technique analytique en raison de la lourdeur de sa procédure d'exécution, ne paraît pas automatisable dans des conditions d'exploitation industrielle. Il en résulte que, en cas de surcharge organique d'un réacteur, le technicien chargé du contrôle de ce dernier ne dispose pas de signes rapidement décelables et réellement significatifs d'un disfonctionnement du réacteur.

En conséquence, la présente invention s'est fixée pour objectif d'apporter un nouveau procédé de régulation et de conduite automatique d'un réacteur de dépollution d'eaux résiduaires par fermentation méthanique assurant une exploitation automatique du réacteur anaérobie tout en optimisant son fonctionnement.

L'idée à la base de la présente invention consiste à mesurer en permanence trois paramètres, non pas dans la phase liquide du réacteur comme dans la technique antérieure, mais dans la phase gazeuse des digesteurs.

En 1988, (RENARD et al. "Adaptative control of anaerobic digestion processes : A pilot scale application". Biotechnology and Bioengineering, vol. 31, pages 287-294), on a tenté de conduire de façon automatique un réacteur de méthanisation de laboratoire, la conduite étant basée sur la seule mesure en continu de la production de gaz. Toutes les deux heures, un dosage de la DCO à l'entrée et à la sortie du réacteur était nécessaire, ce qui, ainsi qu'on l'a vu précédemment, ne peut être réalisable sur un site industriel.

En 1989, (MOSEY et al. "Patterns of hydrogen in biogas from the anaerobic digestion of milk-sugars". Water Science Technology, vol. 21, pages 187-196), on s'est interrogé sur l'opportunité de mesurer en continu l'hydrogène du biogaz pour régulariser le débit de la pompe d'alimentation en eau brute. Le réacteur testé était alimenté en semi-continu, ce qui impliquait des temps de séjours de 21 jours non compatibles avec la réalité industrielle. Les réponses de la mesure de l'hydrogène dans le gaz semblaient insuffisantes et pas toujours utiles pour conduire de façon efficace un réacteur de méthanisation. D'autres auteurs ont tenté de mesurer en parallèle la production de gaz, la teneur en CH₄, en CO₂ et en hydrogène.

Cependant, (SLATER et al. "A microcomputer-based instrumentation system for anaerobic waste water treatment processes". Water Research, 1990, vol. 24, N° 1, pages 121-123), on n'a pas détecté de variations sensibles et utilisables des teneurs en CH₄ et CO₂ et on n'a donc pas tenté de corréler ces paramètres entre eux.

D'autre part, dans une étude faisant le point sur tous les procédés de conduite de fermenteurs (SWIZENBAUM et al. "Monitoring of the anaerobic methane fermentation process". Enzyme Microbiology and Technology, 1990, vol. 12, pages 722-730), on trouve un bilan négatif en ce qui concerne l'utilisation des paramètres de type production de gaz, teneurs en CH₄, CO₂ et H₂.

Contrairement à tous ces auteurs qui ont comparé la réponse de chaque paramètre pris individuellement, les présents titulaires se sont intéressés à la combinaison de ces paramètres. Sur un pilote alimenté avec une eau brute industrielle, on a simulé l'ensemble des cas de surcharges ou d'accidents pouvant survenir sur une installation industrielle. Chaque cas a été caractérisé par une combinaison de réponses issue de la mesure simultanément de trois paramètres : production de gaz, rapports CH₄/CO₂ et teneur en H₂ du biogaz.

La présente invention a donc pour objet un procédé de régulation et de conduite automatique d'un dispositif de dépollution d'eaux résiduaires par fermentation méthanique, caractérisé en ce qu'il consiste à mesurer simultanément, dans la phase gazeuse des digesteurs d'un réacteur de fermentation, les trois paramètres suivants : le débit de gaz issu de la transformation de la matière organique lors de la fermentation, le rapport des pourcentages de méthane et de gaz carbonique et la teneur en hydrogène gazeux, puis à traiter en temps réel les informations ainsi recueillies afin d'obtenir des signaux traduisant l'état instantané de l'écosystème du dispositif de dépollution.

Selon un mode de mise en oeuvre du procédé objet de l'invention, les mesures des trois paramètres sont effectuées à partir de capteurs qui sont disposés sur la conduite recueillant le biogaz produit en sortie du réacteur. Selon une variante, ces capteurs peuvent être disposés sur une boucle de recyclage de biogaz.

Selon l'invention, les signaux ainsi obtenus à partir de la mesure des trois paramètres mentionnés ci-dessus peuvent être utilisés pour actionner une alarme ou bien pour agir directement sur le système d'alimentation en eaux résiduaires du réacteur.

On comprend que le procédé objet de la présente invention s'écarte délibéremment de la technique utilisée jusqu'à présent sur site industriel, étant donné qu'à l'heure actuelle, le volume journalier cumulé de biogaz et le rapport méthane/gaz carbonique étaient considérés comme des paramètres de production, c'est-à-dire comme des paramètres rendant compte de l'état final de la réaction. Or, dans le procédé objet de la présente invention et tel que défini ci-dessus, ces paramètres sont considérés commen rendant compte instantanément de la réaction qui se produit dans les réacteurs utilisés de façon industrielle et fonctionnant selon le mode infiniment mélangé, le gaz produit, recueilli en sortie du réacteur étant une image fidèle du gaz confiné au sein du mélange réactionnel.

Par conséquent, les paramètres mesurés selon le procédé de l'invention : débit de gaz, rapport des pourcentages méthane/gaz carbonique et hydrogène gazeux traduisent en temps réel l'état de l'écosystème ainsi que son évolution. Cette combinaison de paramètres permet de dépasser la notion de facteur global de fermentation pour pénétrer en profondeur l'état physiologique et le métabolisme de la biomasse. La mesure de ces trois paramètres et le traitement des informations ainsi recueillies permettent de déceler, très en amont du système, un problème qui ne serait pas encore détecté en utilisant la technique antérieure mentionnée ci-dessus, c'est-à-dire la mesure des paramètres classiques pH, DCO et alcalinité. Cette détection en temps réel d'un disfonctionnement du réacteur permet de prendre immédiatement des mesures pour corriger ces problèmes, ce qui assure une conduite et une régulation automatiques du dispositif.

On a donné ci-après un certain nombre d'exemples indiquant comment différents facteurs peuvent modifier l'écosystème du réacteur et donc perturber le fonctionnement des fermentations anaérobies. Dans ces exemples, qui ne présentent bien entendu aucun caractère limitatif, on a choisi les trois facteurs suivants :
1° une surcharge organique provoquée par une augmentation de la concentration de l'effluent à traiter ;
2° une baisse de température du réacteur et ;
3° une modification de la nature de la pollution de l'eau résiduaire traitée.

Au cours de cette description on se réfère aux dessins annexés, sur lesquels ;
- les figures 1a à 1c sont des courbes illustrant l'évolution des paramètres mesurés selon le procédé de l'invention, lors d'une surcharge organique du réacteur (exemple 1) ;
- les figures 2b à 2d sont des courbes illustrant l'évolution des paramètres mesurés selon le procédé de l'invention lors d'une baisse de la température du réacteur selon la courbe de la figure 2a (exemple 2) ;
- les figures 3a à 3c sont des courbes illustrant l'évolution des paramètres mesurés selon le procédé de l'invention lors d'une modification de la nature de la pollution de l'eau brute traitée (exemple 3) ;
- La figure 4 est une représentation schématique d'un réacteur mettant en oeuvre le procédé objet de la présente invention et ;
- La figure 5 est un organigramme illustrant un exemple de logiciel utilisé pour l'exploitation du procédé selon la présente invention.

### EXEMPLE 1

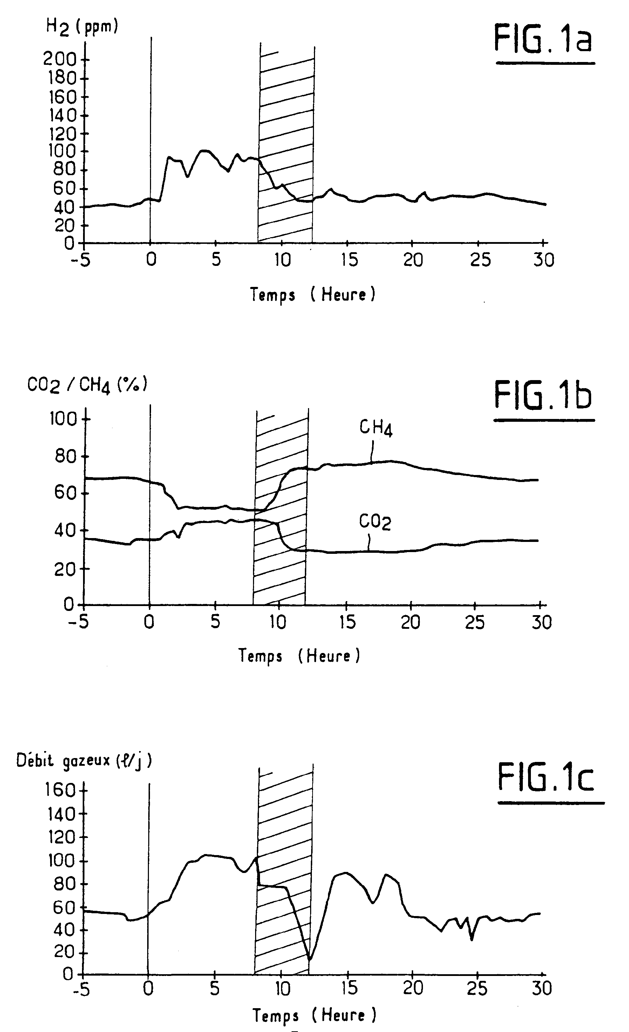

Ce premier exemple se réfère à un réacteur dont l'alimentation normale est de 20g par litre en DCO et auquel on applique une surcharge organique, provoquée par une augmentation d'un facteur 6 de la concentration de l'effluent à traiter, sur une période de huit heures. Lors de cet exemple, l'alimentation a été arrêtée de 8 heures à 12 puis remise en service dans les conditions normales de fonctionnement. Sur la figure 1a, on a représenté la courbe de la teneur en hydrogène gazeux (en ppm) en fonction du temps, sur la figure 1b, on a représenté les variations du rapport pourcentage de gaz carbonique/pourcentage de méthane en fonction du temps et sur la figure 1c,on a représenté la courbe de variation du débit gazeux (en litres par jour) en fonction du temps. L'examen de ces courbes montre qu'il se produit déjà une évolution des trois paramètres une heure après le début de la surcharge :
- l'hydrogène gazeux passe d'une valeur stable de 40 ppm à une valeur plafond de l'ordre de 100 ppm (soit une augmentation de 150%) ;
- le rapport des pourcentages gaz carbonique/méthane dans le même temps varie par suite d'une baisse de la teneur en méthane et d'une augmentation de la teneur en gaz carbonique ;
- la variation brusque des deux paramètres ci-dessus est suivie par une évolution à la baisse de la production de gaz, trois heures après le début de l'apparition de la surcharge organique.

On constate donc que grâce à l'invention on obtient des conditions reproductibles et significatives d'une situation instable de l'écosystème et, la mesure des trois paramètres mentionnés ci-dessus et le traitement des signaux obtenus à partir des informations recueillies à partir de cette mesure permettent de déclencher une intervention sur le réacteur laquelle, ainsi qu'on l'a précisé ci-dessus et comme on le verra ci-après, peut consister en le déclenchement d'une alarme ou, dans un système intégré de régulation, en une intervention immédiate sur le débit d'alimentation en eau brute du réacteur.

### EXEMPLE 2

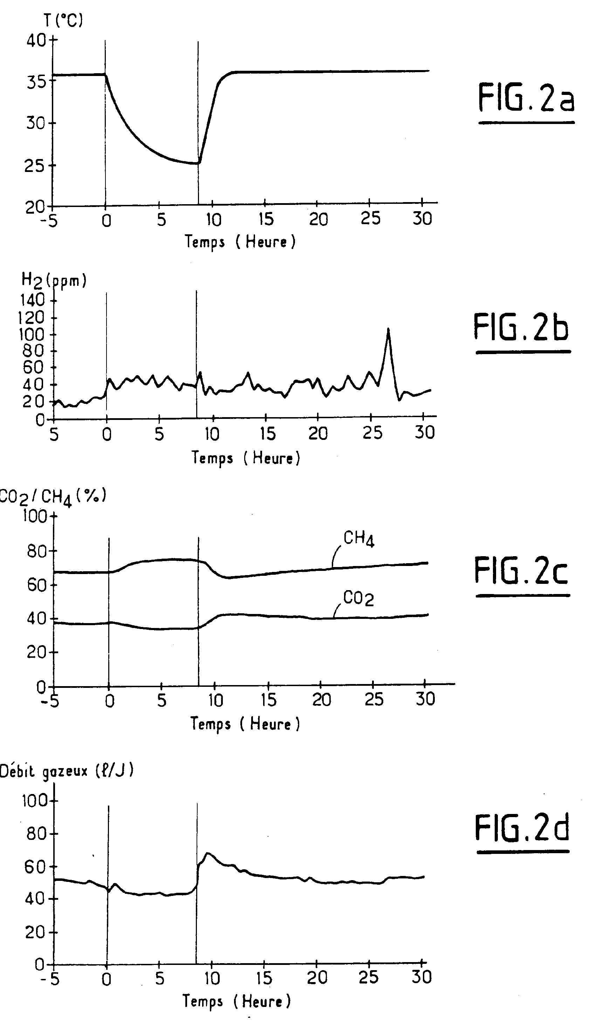

Cet exemple concerne le cas d'une baisse de température du réacteur conformément à la figure 2a. Les figures 2b, 2c et 2d correspondent aux figures 1a, 1b et 1c de l'exemple précédent et elles montrent donc l'évolution en fonction du temps des trois paramètres mesurés conformément au procédé de l'invention :
- on constate (figure 2b) que l'augmentation de la teneur en hydrogène gazeux est plus faible que dans le cas de l'exemple 1, c'est-à-dire lors d'une augmentation de la concentration de l'effluent à traiter ;
- les teneurs en méthane et en gaz carbonique évoluent dans le sens opposé c'est-à-dire que, dans cet exemple,la teneur en méthane augmente alors que la teneur en gaz carbonique diminue ;
- le débit tend à diminuer, ce qui traduit bien une diminution de l'activité bactérienne.

Comme dans l'exemple précédent, la mesure de ces trois paramètres permet de détecter immédiatement un mauvais fonctionnement du réacteur et de prendre sans tarder les mesures permettant ce disfonctionnement.

### EXEMPLE 3

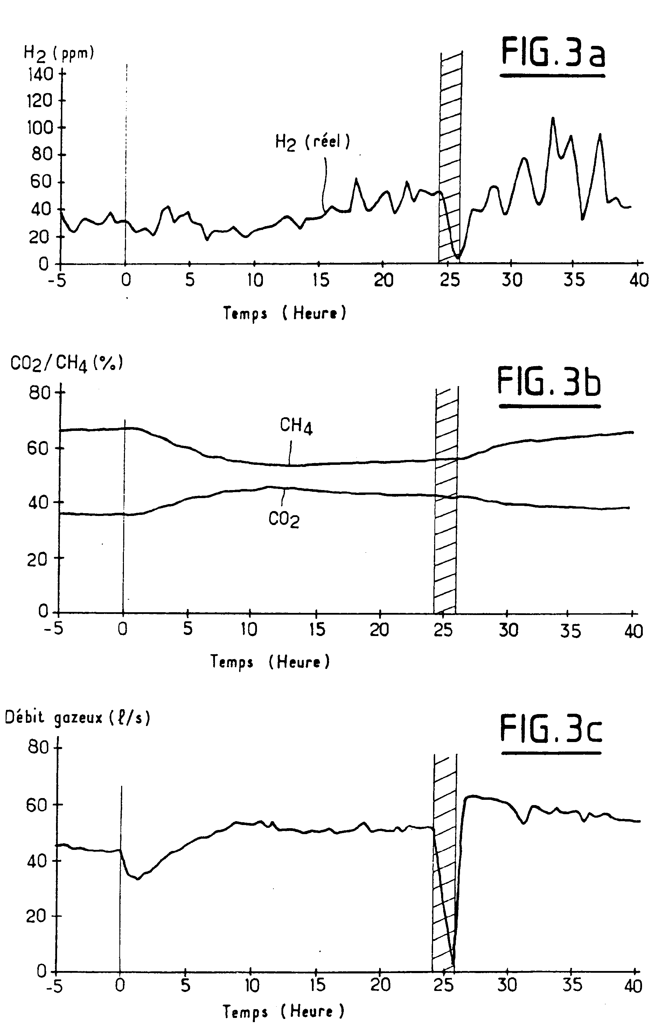

Dans ce troisième exemple, on a illustré la situation qui se présente lors d'un changement de nature de la pollution, c'est-à-dire de l'effluent traité. Dans cet exemple, jusqu'à 0 heure, l'alimentation en effluents était constitué par de la vinasse (en une concentration de 20 g par litre en DCO) puis de 0 à 24 heures le réacteur était alimenté en glucose selon la même concentration, l'alimentation était arrêtée de 24 à 26 heures pour être ensuite reprise à l'aide de vinasses. Le traitement d'un tel problème demande une adaptation brutale des bactéries au nouveau substrat et, dans certains cas, nécessite un développement d'un nouveau type de bactéries. La nécessaire variation du métabolisme de la biomasse s'est traduite également par une évolution des trois paramètres mesurés par l'invention, évolution qui est illustrée par les figures 3a à 3c qui correspondent aux figures 1a à 1c :
- on constate que la teneur en hydrogène gazeux (figure 3a) ne semble augmenter que très tardivement après le début de l'arrivée d'un nouveau substrat ;
- par contre, on remarque que le débit gazeux subit instantanément une chute brutale liée à l'adaptation des bactéries puis l'on constate que la production de gaz se rétablit et qu'elle a même tendance à augmenter par rapport au substrat initial ;
- la variation du rapport des pourcentages de gaz carbonique/méthane est très rapide comme dans les deux cas précécents.

On peut donc, comme dans les exemples précédents, déceler immédiatement la modification de fonctionnement du réacteur et prendre les mesures nécessaires à un retour à un mode de fonctionnement normal.

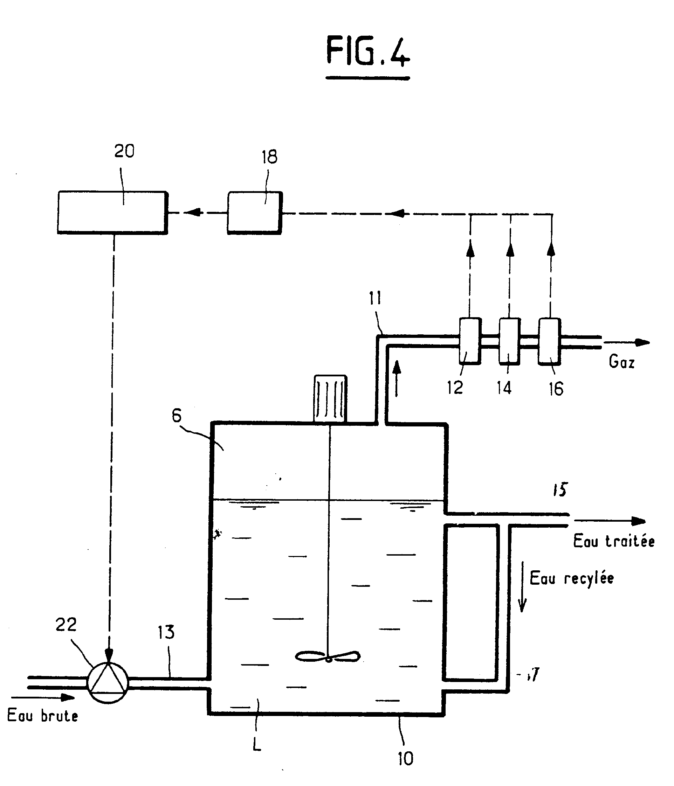

On se réfère maintenant à la figure 4 sur laquelle on a représenté de façon schématique un exemple de réalisation d'un réacteur mettant en oeuvre le procédé objet de l'invention tel que spécifié ci-dessus.

Sur cette figure on voit en 10 le réacteur en fonctionnement avec sa phase liquide L et sa phase gazeuse G. L'effluent est amené à la base du réacteur par une conduite 13, cette alimentation étant contrôlée à l'aide d'une pompe 22 et l'eau traitée est évacuée par une conduite 15 sur laquelle est montée une conduite 17 pour le recyclage dans le réacteur d'une portion de l'eau traitée. La référence 11 désigne la conduite de sortie de biogaz sur laquelle, selon l'invention, on positionne des capteurs 12, 14, 16 permettant de relever en temps réel des informations concernant respectivement les trois paramètres mesurés selon le procédé de l'invention : débit de gaz issu de la transformation de la matière organique lors de la fermentation ; rapport des pourcentages de méthane et de gaz carbonique et teneur en hydrogène gazeux. Les informations ainsi recueillies peuvent être traitées dans une unité centrale de traitement 18 et à partir de cette unité les signaux obtenus peuvent être utilisés pour agir sur un système de commande 20 de la régulation de la pompe 22 d'alimentation en eau résiduaire. Ainsi, dans ce mode de mise en oeuvre particulier et non limitatif du procédé de l'invention, on peut réaliser une régulation bouclée. Selon un autre mode de mise en oeuvre les informations recueillies à partir des capteurs 12, 14, 16 peuvent être utilisées pour actionner une alarme, ce qui permet alors aux techniciens chargés du fonctionnement du réacteur de prendre les mesures nécessaires pour assurer un retour aux conditions normales de fonctionnement.

Grâce à l'invention, les trois paramètres sont mesurés en continu sur la sortie de biogaz du réacteur et les capteurs utilisés pour obtenir les informations concernant l'évolution de ces trois paramètres présentent la particularité de ne pas se dérégler étant donné qu'ils sont placés dans une phase non-aqueuse et ne contenant pas de biomasse, ce qui évite tout risque de contamination et de dérive des signaux obtenus. Ainsi l'invention offre à l'exploitant un moyen fiable et sans entretien de contrôle du fonctionnement d'une station d'épuration.

On notera que les capteurs peuvent être disposés sur une boucle de recyclage de biogaz.

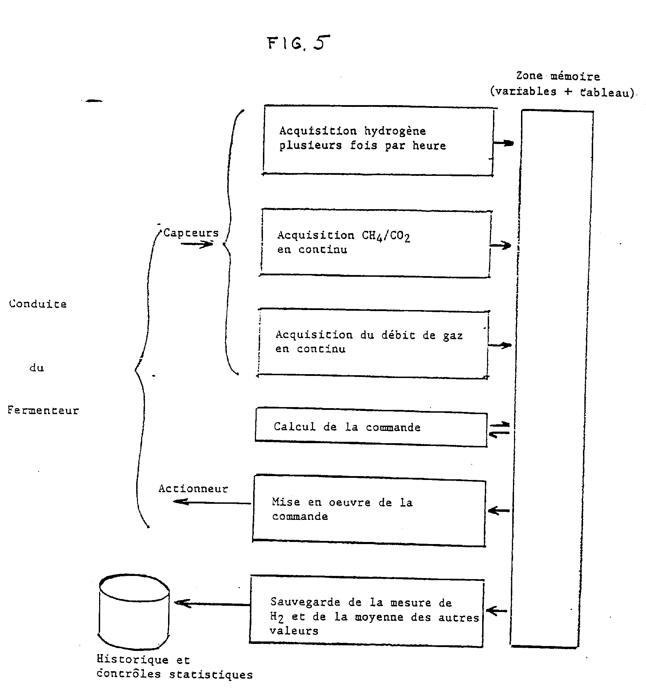

On se réfère maintenant à la figure 5 qui illustre l'organigramme d'un logiciel pouvant être utilisé dans le traitement des informations recueillies à partir des capteurs mis en oeuvre par l'invention.

Ce logiciel, développé dans un langage de programmation multitâches, est supporté par un système d'exploitation en temps réel, les algorythmes des différents capteurs, ainsi que le calcul des paramètres de la commande mise en oeuvre, imposant dans ce procédé, une programmation multiprocessus.

Les acquisitions se font à partir des capteurs placés dans le circuit de gaz. Deux paramètres sont mesurés en continu : rapport des pourcentages méthane/gaz carbonique et débit gazeux. Sur une série de mesures effectuées à intervalle donné, déterminé par le système biologique et choisi de façon à ce qu'il soit compatible avec le domaine d'application, on calcule la moyenne puis on compare ensuite les moyennes de chaque intervalle. La mesure de la teneur en hydrogène gazeux est effectuée de façon discontinue à intervalles réguliers. On compare des valeurs successives. Les acquisitions sont stockées dans une zone mémoire commune aux différentes tâches et sauvegardées plusieurs fois par heure. A partir de ces données, et en parallèle avec les acquisitions on effectue en continu le calcul des paramètres d'une commande optimale et sa mise en oeuvre sur l'alimentation du réacteur, ou sur une alarme, comme indiqué ci-dessus, en cas de disfonctionnement du système ou pour agir sur d'autres moyens ou paramètres utilisés de façon classique dans ce type d'application.

## Revendications

1. Procédé de régulation et de conduite automatique d'un dispositif de dépollution d'eaux résiduaires par fermentation méthanique, caractérisé en ce qu'il consiste à mesurer simultanément, dans la phase gazeuse des digesteur d'un réacteur de fermentation les trois paramètres suivants : le débit de gaz issu de la transformation de la matière organique lors de la fermentation, le rapport des pourcentages de méthane et de gaz carbonique et la teneur en hydrogène gazeux, puis à traiter en temps réel les informations ainsi recueillies afin d'obtenir des signaux traduisant l'état instantané de l'écosystème du dispositif de dépollution.

2. Procédé selon la revendication 1, caractérisé en ce que la mesure des trois paramètres est utilisée pour actionner un moyen d'alarme.

3. Procédé selon la revendication 1, caractérisé en ce que la mesure des trois paramètres est utilisée pour agir directement sur le système de l'alimentation en eau résiduaire du réacteur.

4. Procédé selon l'une quelconque des revendications précédentes, caractérisé en ce que les mesures des trois paramètres sont effectuées à partir de capteurs qui sont disposés sur la conduite de biogaz à la sortie du réacteur.

5. Procédé selon l'une quelconque des revendications précédentes, caractérisé en ce que les mesures des trois paramètres sont effectuées à partir de capteurs qui sont disposés sur une boucle de recyclage du biogaz.

## Patentansprüche

1. Verfahren für die Regelung und selbsttätige Steuerung einer Vorrichtung zur Abwasserreinigung durch Methangärung, **dadurch gekennzeichnet, daß** in der Gasphase der Digerierapparate eines Gärungsreaktors die drei folgenden Parameter gleichzeitig gemessen werden: die Menge des von der Umwandlung des organischen Stoffes bei der Gärung entstehenden Gases, das Verhältnis des Prozentgehaltes an Methan und an Kohlendioxid und der Gehalt an gasförmigem Wasserstoff, und daß dann in Echtzeit die so erhaltenen Informationen verarbeitet werden, um Signale zu erhalten, die den Momentanzustand des Ökosystems der Reinigungsvorrichtung umsetzen.

2. Verfahren nach Anspruch 1, **dadurch gekennzeichnet, daß** die Messung der drei Parameter zur Betätigung einer Alarmeinrichtung verwendet wird.

3. Verfahren nach Anspruch 1, **dadurch gekennzeichnet, daß** die Messung der drei Parameter verwendet wird, um direkt auf das Abwasser-Zufuhrsystem des Reaktors zu wirken.

4. Verfahren nach einem der vorhergehenden Ansprüche, **dadurch gekennzeichet, daß** die Messung der drei Parameter von Sensoren ausgehend durchgeführt wird, die auf der Biogasleitung am Ausgang des Reaktors angeordnet sind.

5. Verfahren nach einem der vorhergehenden Ansprüche, **dadurch gekennzeichnet, daß** die Messung der drei Parameter von Sensoren ausgehend durchgeführt wird, die auf einem Biogas-Rückführungskreislauf angeordnet sind.

## Claims

1. Process for controlling and automatically operating an apparatus for waste water purification by methane fermentation, characterized in that the process consists of simultaneously measuring, in the gaseous phase of the digester of a fermentation reactor, the following three parameters: the production rate of gas resulting from the conversion of the organic material during the fermentation, the ratio of the percentages of methane and carbon dioxide and the gaseous hydrogen content, then of processing in real time the information thus acquired in order to obtain signals representing the instantaneous state of the eco-system of the purification apparatus.

2. Process according to Claim 1, characterized in that the measurement of the three parameters is used for actuating an alarm means.

3. Process according to Claim 1, characterized in that the measurement of the three parameters is used for acting directly upon the feed system for waste water to the reactor.

4. Process according to any one of the preceding Claims, characterized in that the measurements of the three parameters are performed from pick-ups which are disposed on the biogas duct at the outlet from the reactor.

5. Process according to any one of the preceding Claims, characterized in that the measurements of the three parameters are performed from pick-ups which are disposed on a loop for recycling the biogas.
